(19) Europäisches Patentamt / European Patent Office / Office européen des brevets

(11) **EP 3 957 240 B1**

(12) **EUROPEAN PATENT SPECIFICATION**

(45) Date of publication and mention
of the grant of the patent:
**06.11.2024 Bulletin 2024/45**

(21) Application number: **19924857.6**

(22) Date of filing: **15.04.2019**

(51) International Patent Classification (IPC):
***A61B 5/053*** (2021.01)  ***A61B 5/02*** (2006.01)
***A61B 5/00*** (2006.01)

(52) Cooperative Patent Classification (CPC):
**A61B 5/02; A61B 5/053; A61B 5/7228**

(86) International application number:
**PCT/CN2019/082729**

(87) International publication number:
**WO 2020/210949 (22.10.2020 Gazette 2020/43)**

(54) **NON-INVASIVE METHOD AND SYSTEM FOR DETECTING FEATURE INFORMATION OF IN VIVO TISSUE**

NICHTINVASIVES VERFAHREN UND SYSTEM ZUR DETEKTION VON MERKMALSINFORMATIONEN VON IN-VIVO-GEWEBE

PROCÉDÉ ET SYSTÈME NON INVASIFS POUR DÉTECTER DES INFORMATIONS DE CARACTÉRISTIQUE D'UN TISSU IN VIVO

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**23.02.2022 Bulletin 2022/08**

(73) Proprietor: **Msheaf Health Management
Technologies Limited
Kowloon, Hong Kong 999077 (CN)**

(72) Inventors:
• **YI, Cheng
Hong Kong 999077 (CN)**
• **WANG, Ling
Hong Kong 999077 (CN)**

• **HE, Bixia
Hong Kong 999077 (CN)**
• **XIE, Peng
Hong Kong 999077 (CN)**

(74) Representative: **Winter, Brandl - Partnerschaft
mbB
Alois-Steinecker-Straße 22
85354 Freising (DE)**

(56) References cited:
| EP-A2- 1 138 259 | WO-A1-2011/158166 |
|---|---|
| CN-A- 102 008 302 | CN-A- 104 783 792 |
| CN-A- 107 767 957 | CN-A- 107 788 983 |
| CN-A- 110 432 903 | US-A1- 2008 009 757 |
| US-A1- 2010 049 077 | US-A1- 2018 067 063 |
| US-A1- 2018 206 759 | US-B1- 6 339 722 |

**Description**

**TECHNICAL FIELD**

The present invention relates to a non-invasive method and system to detect characteristic information of body tissues.

**BACKGROUND**

Bio-impedance and bio-reactance measurements as non-invasive methods to measure blood flow and body fluid levels have been widely explored. These techniques are well accepted in the medical field. However, they also suffer some drawbacks. First, all the computed parameters are based on impedance, which is frequency dependent. These parameters can only indirectly represent the cardiovascular state. Moreover, since the parameters are frequency dependent, they will suffer frequency-selectivity impairment. Second, the connecting tissue's impedance plays an important role in the impedance measurements. Traditional bio-impedance and bio-reactance measurements are a mixed result of the surrounding tissues' impendence and the target tissue's impedance; however, sometimes, it can be difficult to determine which impedance dominates. Therefore, the mixed impedance varies with individuals; even for the same person, the mixed impedance varies with different tissues' states. Therefore, bio-impedance and bio-reactance are not good candidates to represent the characteristics of body fluid and cardiovascular circulation.

From the electrical perspective, bio-tissues are characterized as conductors and non-conductors. Conductors are measured by the conductance (reciprocal of resistance), while non-conductors can be measured by capacitance or permittivity. One widely recognized human tissue model is the Cole model. Basically, the alternating current is mainly conducted by the extracellular liquid, which is mainly resistance at low-frequencies, such as 1KHz. As the alternating current's frequency increases, the alternating current passes through both extracellular liquid and cells. Since cells have membranes which function like capacitors, the alternating voltage will have phase change. As the frequency keeps increasing to be more than 1 MHz, the cells' membrane effects become insignificant in the total impedance, and the total impedance becomes a pure resistor again. The Cole model describes this behavior.

Any tissue's change will basically cause changes in its resistance (or conductance) and capacitance. Therefore, to present tissues' changes, the measurement of the tissues' resistance and capacitance changes are far more reliable than the measurement of the mixed bio-impedance and bio-reactance, which includes the connecting tissue's impedance and reactance. Since the tissues' conductance and capacitance are frequency dependent, a frequency band has to be selected. It is generally believed that the tissues' information is mainly within a 10KHz to 1MHz band. Accordingly, to measure the tissue's conductance and capacitance, multi-frequency alternating stimuli (electrical currents) in the 10 KHz to 1 MHz band is used. (Inverse) Fast Fourier Transform (IFFT/FFT) or Orthogonal Frequency Division Multiplexing (OFDM) method is a common method for generating and demodulating synchronized multiple frequencies. They can control the phase at each frequency and adjust the same arbitrarily, so that it is better than quasi-multi-frequency synchronization signals generated by random sequences. According to Ohm's law, the tissue's conductance and capacitance can be computed from the multiple-frequency alternating currents.

WO 2011/158166 A1 discloses systems and methods for measurements of anatomical parameters in a region of interest in a subject. A method of using electrical measurements, such as voltage or current, across a range of frequencies derived from data representative of the voltage signals is also discussed. These measurements are used to determine parameters of one or more anatomical features of the subject.

US 2018/0206759 A1 discloses a method for use in determining fluid levels within a subject. The method includes, in a processing device, determining at least one impedance value measured for the subject, determining physical dimensions for at least part of at least one segment of the subject, using the physical dimensions to determine a shape factor at least partially indicative of a shape of the at least one segment and calculating a fluid indicator indicative of the fluid levels in the segment at least in part using the at least one impedance value and the shape factor.

Further related prior art is disclosed in US 2018/0067063 A1, EP 1 138 259 A2 and US 6 339 722 B1.

**SUMMARY OF THE INVENTION**

In order to solve the problems in the prior art, the present invention provides a non-invasive method and system according to the independent claims. The method is mainly used for the health state monitoring of organisms, the elasticity measurement and verification of a cardiovascular system, and the detection of information for non-therapeutic purposes.

To achieve the purpose above, the present disclosure provides a technical solution:
A non-invasive method to detect characteristic information of body tissues is disclosed. The method is used to capture changes in body fluids, blood flow, and/or cardiovascular circulatory tissues. The method comprises: generating multiple synchronous alternating currents at different frequencies with characteristics of OFDM symbols by using IFFT, and transmitting them into a human or animal body; receiving the alternating currents modulated by tissues and their changes in a human or animal body; amplifying the modulated alternating currents and digitally converting them into digital signals; and pre-processing the digital signals, the pre-processing comprising: segmenting the digital signals into OFDM symbol sequences, demodulating the OFDM symbol sequences by using FFT to obtain frequency domain signals; estimating the state of a target tissue based on the frequency domain signals.

Preferably, the generating multiple synchronous alternating currents at different frequencies simultaneously by using IFFT or OFDM comprises: generating multiple synchronous alternating currents at different frequencies from the frequency domain to the time domain. The alternating currents at different frequencies are periodic, and the intensity, phase, and/or frequency of the alternating currents are adjustable.

Preferably, the receiving the alternating currents modulated by tissues and their changes in a human or animal body comprises: determining the period of the alternating currents and receiving the modulated alternating signals synchronously in each of the periods.

The estimating the state of the target tissue based on frequency domain signals comprises: separating the frequency domain signals and filtering the separated frequency domain signals; calculating resistances and capacitances based on the frequency domain signals; and estimating the state of the target tissue by using the resistance and capacitance values.

Preferably, the separating the frequency domain signals comprises: based on the complex impedance of the frequency domain signals, calculating a human body system transfer function through system identification and channel estimation methods.

Preferably, the calculating the resistance and the capacitance values of the target tissue and peripheral tissues comprises: calculating the resistance and capacitance values of the target tissue and the peripheral tissues based on the human body system transfer function.

According to the invention, estimating the state of the target tissue based on the resistance and capacitance values comprises: performing multi-chamber modeling by using the resistance and capacitance values, where each chamber consists of a resistor and a capacitor connected in parallel and the multiple chambers are connected in series, or in parallel, or in a manner of combination of serial and parallel connections.

Preferably, the performing multi-chamber modeling can be two-chamber modeling, wherein a connecting tissue is between an electrode and the target tissue.

Preferably, the frequency range is from 10KHz to 1MHz .

In order to achieve the purpose above, the present disclosure provides a technical solution:

A system to implement any of the methods above is provided, the system comprising a terminal and a processor, wherein the terminal comprises: a generator, configured to use IFFT to generate multiple synchronous alternating currents at different frequencies with characteristics of OFDM symbols; one or more sensors, configured to transmit multiple alternating currents into a human body or animal body and receive the alternating currents modulated by tissues' changes in the human or animal body; one or more amplifiers, configured to amplify the modulated alternating currents and digitally convert them into digital signals; and a pre-processing module, which segments the input digital signals into OFDM symbol sequences by using the characteristics of the OFDM symbols and demodulates the OFDM symbols by FFT to obtain the frequency domain signals; wherein the processor is used for calculating resistances and capacitances and estimating the state of the target tissue by using the resistance and capacitance values.

Preferably, the generator is used to generate the multiple alternating currents at different frequencies from the frequency domain to the time domain, i.e. OFDM symbols, wherein the alternating currents at different frequencies are periodic, i.e. the OFDM symbols are output repetitively. The intensity, phase and/or frequency of the alternating currents are adjustable.

Preferably, the processor is used to determine the period of the alternating currents, i.e. the length of the OFDM symbols, and to receive the modulated AC signals synchronously at each period.

Preferably, the sensor is used to collect single or multiple pieces of data from different sites.

The pre-processing module is further used to separate the frequency domain signals, and to filter the separated frequency domain signals.

Preferably, the system further comprises an accelerator, the accelerator is used to calculate the resistances and the capacitances based on the frequency domain signals, and the calculation comprises the use of system identification or channel estimation procedure.

According to the invention, the Ute processor establishes an equivalent circuit of multiple chambers based on the resistance and capacitance values, where each chamber consists of a resistor and a capacitor connected in parallel, and multiple chambers are connected in series, or in parallel, or in a manner of combination of serial and parallel connections.

Preferably, the system may comprise a database for storing processing results and data of the processor, and the processor can retrieve the database.

Preferably, the processor can be remote and remotely observe the system working in a real-time mode.

Preferably, the terminal further comprises a human-machine interface to operate the system and/or display results.

The invention is set out in the appended claim set. The present invention relates to a non-invasive method and system to detect characteristic information of body tissues. Compared to the prior art, it applies multiple synchronous periodic alternating currents at different frequencies to the human body simultaneously. After receiving the signals modulated by the body tissues, the information from the cardiovascular system and the surrounding tissues is demodulated and extracted from carriers at specified frequencies. Combined information of the cardiovascular circulatory system and surrounding tissues is obtained through the implementation of system identification or channel estimation procedures, and calculates the resistances and capacitances of the cardiovascular system and the surrounding tissues from the system transfer function to distinguish the cardiovascular system from its surrounding tissues. The calculated resistances and capacitances are utilized to represent the state of body fluid and cardiovascular circulatory tissues. Thus, the corresponding state information can be accurately and reliably acquired, and accurate measurement of the target tissue can be achieved

## BRIEF DESCRIPTION OF THE DRAWINGS

The present invention will be further described below in conjunction with the drawings and embodiments:

FIG. 1 is a schematic diagram of a terminal system according to a preferred embodiment of the present invention;
FIG. 2 is a functional or structural diagram of a terminal according to a preferred embodiment of the present invention;
FIG. 3 is a structural diagram of a system according to a preferred embodiment of the present invention;
FIG. 4 is a schematic diagram of a measurement circuit of a multi-chamber model according to a preferred embodiment of the present invention;
FIG. 5 is a schematic diagram of a circuit structure for system test according to a preferred embodiment of the present invention;
FIGS. 6a-6c are schematic diagrams of the time domain signals and frequency responses of the system test on measuring a resistor network according to a preferred embodiment of the present invention;
FIGS.7a-7b are oscillograms of 10 synchronous frequency tones under theoretical transmission according to a preferred embodiment of the present invention;
FIG. 8 is a schematic diagram of signals actually received by the system according to a preferred embodiment of the present invention;
FIG. 9 is a schematic diagram of received external signals according to a preferred embodiment of the present invention, namely, a schematic diagram of human body signals;
FIGS. 10a-10b are schematic diagrams of amplitude and phase response of an internal resistor network according to a preferred embodiment of the present invention;
FIGS. 11a-11b are schematic diagrams of the actually measured amplitude and phase response of the human body according to a preferred embodiment of the present invention;
FIGS. 12a-12b are schematic diagrams of the corrected amplitude and phase response of the human body according to a preferred embodiment of the present invention.
FIG. 13 is a schematic diagram of the transfer function after system identification and the human body frequency response according to a preferred embodiment of the present invention;
FIGS. 14a-14c are schematic diagrams of artery results from a two-chamber model on aorta measurement according to a preferred embodiment of the present invention;
FIGS. 15a-15c are schematic diagrams of peripheral results from a two-chamber model on aorta measurement according to a preferred embodiment of the present invention;
FIGS. 16a-16c are schematic diagrams of ventricular results from a two-chamber model on ventricular measurement according to a preferred embodiment of the present invention;
FIGS. 17a-17c are schematic diagrams of peripheral results from a two-chamber model on ventricular measurement according to a preferred embodiment of the present invention.
FIG. 18a-18c are schematic diagrams of artery results from a two-chamber model on upper chest measurement according to a preferred embodiment of the present invention;
FIGS. 19a-19c are schematic diagrams of peripheral results from a two-chamber model on upper chest measurement according to preferred embodiment of the present invention;
FIGS. 20a-20c are schematic diagrams of artery/vein results from a two-chamber model on right lung measurement according to a preferred embodiment of the present invention;
FIGS. 21a-21c are schematic diagrams of peripheral results from a two-chamber model on right lung measurement according to a preferred embodiment of the present invention;
FIGS. 22a-22c are schematic diagrams of artery/vein results from a two-chamber model on left lung measurement according to a preferred embodiment of the present invention;
FIGS. 23a-23c are schematic diagrams of peripheral results from a two-chamber model on left lung measurement according to a preferred embodiment of the present invention;

## DETAILED DESCRIPTION OF EMBODIMENTS

The present invention will now be described in further detail with reference to the drawings. These drawings are all simplified schematic diagrams, which merely illustrate the basic structure of the present invention in a schematic manner and only show the constitution related to the present invention.

The present invention relates to a non-invasive technology to detect electrical properties of tissues in an organism, such as the resistances and capacitances of the tissues and their change patterns. Its goal is to capture changes in body fluid, blood flow and cardiovascular circulatory tissues for organisms' health state monitoring, cardiovascular system elasticity verification, and detection on information for non-therapeuthic purposes.

The method provided by the present invention is used to detect the state of human or animal tissues, including body fluid and blood flow, the states of arteries, heart, and lungs. The method extracts the changes of the tissues' resistances and capacitances in order to obtain quantitative correlation of states of cardiovascular circulation, body fluid and cardiovascular tissues (including heart and lungs). Therefore, an embodiment of the present invention provides a measurement method, at least including:

1. Generating multiple synchronous alternating currents at different frequencies with characteristics of OFDM symbols by using IFFT, and transmitting them into a human or animal body.
Specifically, at a transmitting end, the embodiment uses a digital signal processing technology, such as IFFT or OFDM, to generate multiple alternating currents (ACs) at different frequencies from frequency domain to time domain, also known as the OFDM symbol sequences. The multiple alternating currents at different frequencies are periodic, with the OFDM symbol length as the period, and the multiple alternating currents at different frequencies are simultaneously applied to the human body, wherein the intensity, phase and/or frequency of the alternating currents are adjustable.
2. Receiving alternating currents modulated by tissues' changes of the human or animal body.

Specifically, the embodiment includes, at the receiving end, determining the period of the alternating currents, i.e., the length of the OFDM symbol, and synchronously receiving the modulated AC signals in each period, i.e., the OFDM symbols modulated by the human body. An alternative embodiment includes, finding out the period and start point of the OFDM symbols based on their characteristics, and demodulating every OFDM symbol to obtain frequency domain signals afterwards.

In an alternative embodiment, multiple alternating currents at different frequencies are simultaneously injected into the human body through electrodes, which form circuits with some external electronic components. When the currents are transmitted in the human body, they are adjusted by body tissues and tissues' changes in the circuit; and the receiving circuit and the injection circuit are partially overlapped, so that the signals modulated by the overlapped human tissues can be detected.

3. Amplifying the modulated alternating currents and digitally converting them into digital signals.

In an alternative embodiment, the received alternating currents modulated by the human or animal body can be current signals or converted voltage signals, which are collectively referred to as modulated OFDM symbol sequences. They are converted into digital signals after amplification. In an alternative embodiment, electrocardiogram (ECG) signals are also superposed into modulated signals. The ECG can be separated by low-pass filtering before digital conversion, be amplified separately and be converted into digital format signals for further processing.

4. Pre-processing the digital signals, wherein the pre-processing includes, segmenting the digital signals into OFDM symbol sequences, and demodulating the OFDM symbol sequences by using FFT to obtain frequency domain signals.

Specifically, the embodiment includes, based on the period and the signal properties of the transmitted signals, namely, the period and properties of OFDM symbols, segmenting digital signals into OFDM symbol sequences, and demodulating each OFDM symbol by using FFT to obtain the frequency domain signals.

In an alternative embodiment, the OFDM symbol characteristics are searched in the received digital signals, and the period is known. After finding the characteristic segments, the start point of the OFDM symbol can be determined. Then the received signals become sequences with OFDM symbol as unit. Each OFDM symbol can be demodulated using FFT, and the signals are converted into frequency domain.

5. Estimating the state of a target tissue based on the frequency domain signals.

Specifically, the embodiment includes, separating the frequency domain signals and filtering the separated frequency domain signals; calculating a human tissue system transfer function based on the frequency domain signals, then calculating the tissues' resistances and capacitances from the system transfer function; and estimating the state of the target tissue using the resistance and capacitance values.

In an alternative embodiment, the embodiment includes, simultaneously detecting the real and imaginary parts, or amplitude and phase changes of multiple alternating voltages at different frequencies (also referred to as frequency tones); calculating a human body system transfer function according to the amplitudes and phases of multiple frequency tones, then calculating the human tissues' resistances and capacitances from the parameters in the human body system transfer function.

The embodiment includes, using the calculated resistances and capacitances to represent the states of body fluid and cardiovascular circulatory tissues. Specifically, the resistances and capacitances are used for multi-chamber modeling, wherein each chamber consists of a resistor and a capacitor connected in parallel, and the chambers are connected in series or in parallel, or in a manner of combination of serial and parallel connections.

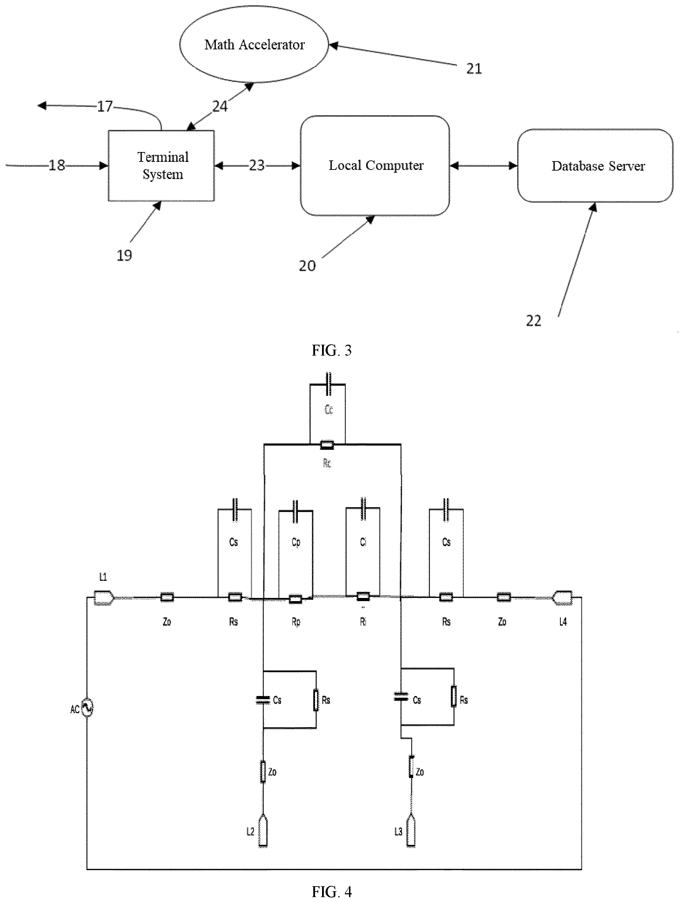

In an alternative embodiment, a two-chamber RC (resistor and capacitor) model is used to simulate the target tissue. A multi-chamber model can be used to simulate the human body. As for thoracic measurement, one chamber represents artery, and/or atria and ventricles, which are the main portions of the cardiovascular circulatory system. The other chamber represents the connecting tissue between an electrode and the cardiovascular circulatory system, where each chamber is represented by a parallel RC network composed of integrated resistor and capacitor. The two chambers are connected in series because the heart or the arterial system is not directly connected to the electrodes. The connecting tissue is always between the measuring electrodes and the heart artery. A system identification or channel estimation technology is used for calculating the integrated R (resistance) and C (capacitance) values. R and C values are used for estimating body fluid, blood flow, and cardiovascular circulatory tissues. The advantage of the two-chamber model is to separate the cardiovascular circulatory system from surrounding tissues. On the basis of the two-chamber model, there is also a three-chamber model, wherein a parallel RC network is connected in parallel with two parallel RC networks connected in series, as shown in FIG. 4. In an alternative embodiment, the three-chamber model is more close to real human tissues, but the calculation amount is large and the stability is poor.

The method according to another embodiment of the present invention includes:

1. Generating multiple synchronous alternating currents at different frequencies with characteristics of OFDM symbols by using IFFT, and transmitting them to a human or animal body.

Specifically, at the transmitting end, this embodiment uses digital a signal processing technology, such as using IFFT to generate OFDM symbols, that is, generating multiple alternating currents at different frequencies from frequency domain to time domain. The multiple alternating currents at different frequencies are periodic, with the period being the length of the OFDM symbol. Besides, multiple alternating currents at different frequencies are simultaneously applied to the human body, wherein the intensity, phase and/or frequency of the alternating currents are adjustable.

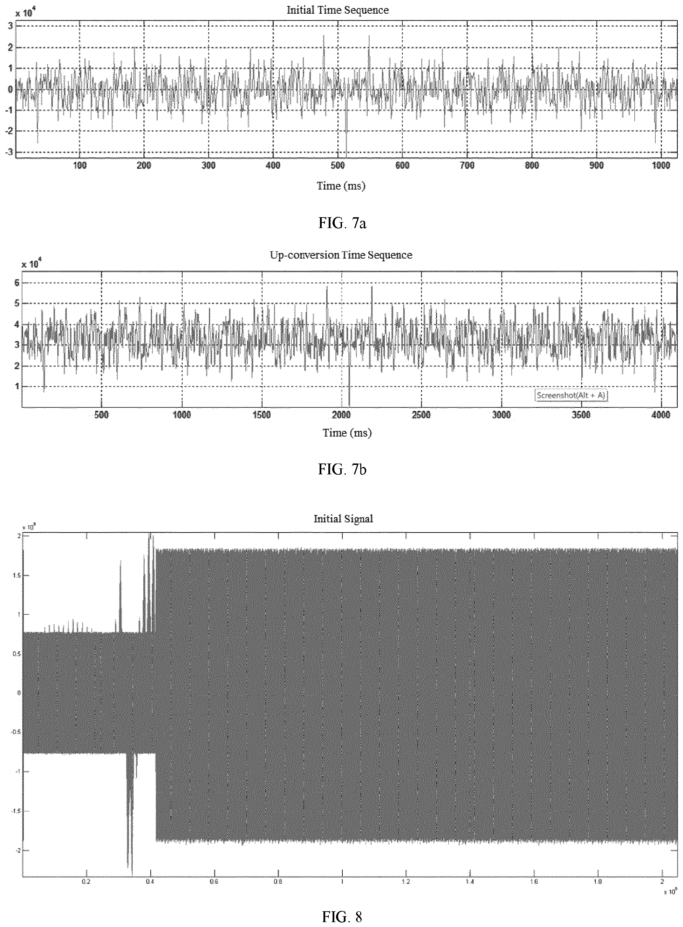

In an alternative embodiment, one period (OFDM symbols) synthesized by these alternating currents is shown in FIGS. 7a and 7b, in which, FIG. 7a is the result (OFDM symbol) of the original time sequence diagram through the IFFT of 1024 points; and FIG. 7b is the result of 4-times original time sequence diagram. In time domain, the sequence repeats indefinitely.

2. Receiving alternating currents modulated by tissues' changes of the human or animal body.

Specifically, the embodiment includes, at the receiving end, determining the period of the alternating currents, i.e. the length of the OFDM symbol, and synchronously receiving the modulated AC signals of the OFDM symbols modulated by the human body in each period. An alternative embodiment includes, finding out the period and start point of the OFDM symbols based on their characteristics, and demodulating every OFDM symbol to obtain frequency domain signals afterwards.

In an alternative embodiment, multiple alternating currents at different frequencies are simultaneously injected into the human body through electrodes, which form circuits with some external electronic components. When the currents circulate in the human body, they are adjusted by body tissues and tissues' changes in the circuit; the receiving circuit and the injection circuit are partially overlapped, so that the signals modulated by the overlapped human tissues can be detected.

In an alternative embodiment, a sequence of millions of points sampled at the receiving end is shown in FIG. 8. The small amplitude part at the front end is the measurement sequence of the internal resistance, and the large amplitude sequence at the back is the human body sequence externally measured. In an alternative embodiment, each measurement uses the internal frequency response to correct the external frequency response for reducing random errors of the system. The large amplitude pulse in the internal resistance is pseudo electrocardiogram data because electrocardiogram signals are combined in high-frequency signals. An alternative embodiment includes, after receiving the modulated voltage signals, finding out the start point of the received OFDM symbol based on a pre-determined system delay, and converting the received signals into an OFDM symbol sequence, i.e., the receiving end is synchronized with the transmitting end.

3. Amplifying the modulated alternating currents and digitally converting them into digital signals.

In an alternative embodiment, the received alternating currents modulated by the human or animal body can be current signals or voltage signals in the receiving circuit. In an alternative embodiment, electrocardiogram (ECG) signals are also superposed into the modulated signals, and the ECC can be separated by low-pass filtering before digital conversion. The embodiment includes, sampling different signals according to different requirements. The sampled signals are modulated and amplified and are converted into digital format signals for further processing.

4. Pre-processing the digital signals, wherein the pre-processing includes, segmenting the digital signals into OFDM symbol sequences, and demodulating the OFDM symbol sequences by using FFT to obtain frequency domain signals.

Specifically, the embodiment includes, based on the period and the signal properties of the transmitted signals, namely, the period and properties of OFDM symbols, segmenting digital signals into OFDM symbol sequences, and demodulating each OFDM symbol by using FFT to obtain the frequency domain signals.

An alternative embodiment includes, demodulating the received synchronous signals. Searching characteristics of the OFDM symbols in the received digital signals, where the period is known. After finding out the characteristic segments, the start point of the OFDM symbol can be determined. Then the received signals become sequences with OFDM symbol as unit. Each OFDM symbol can be demodulated using FFT, and the signals are converted into frequency domain.

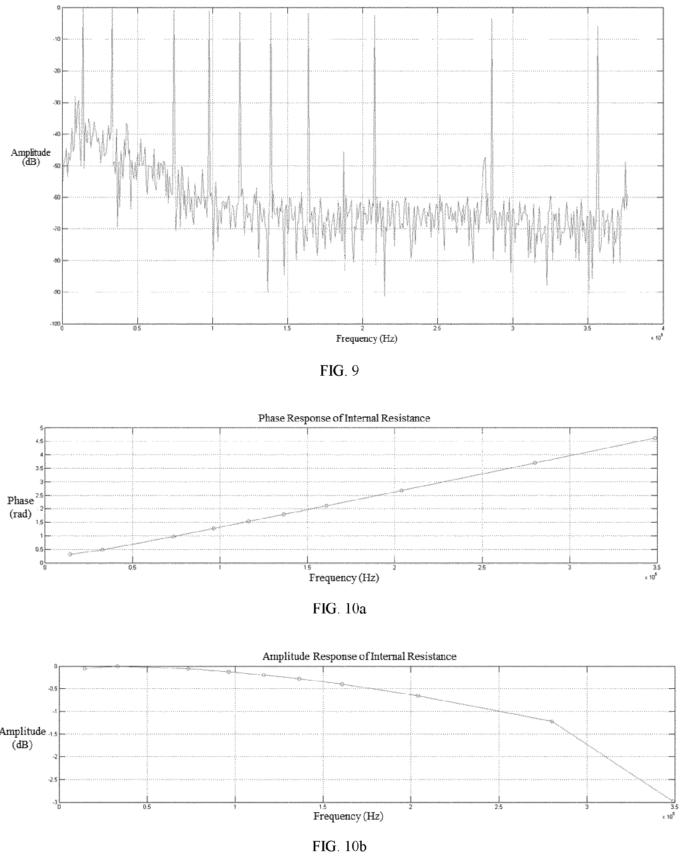

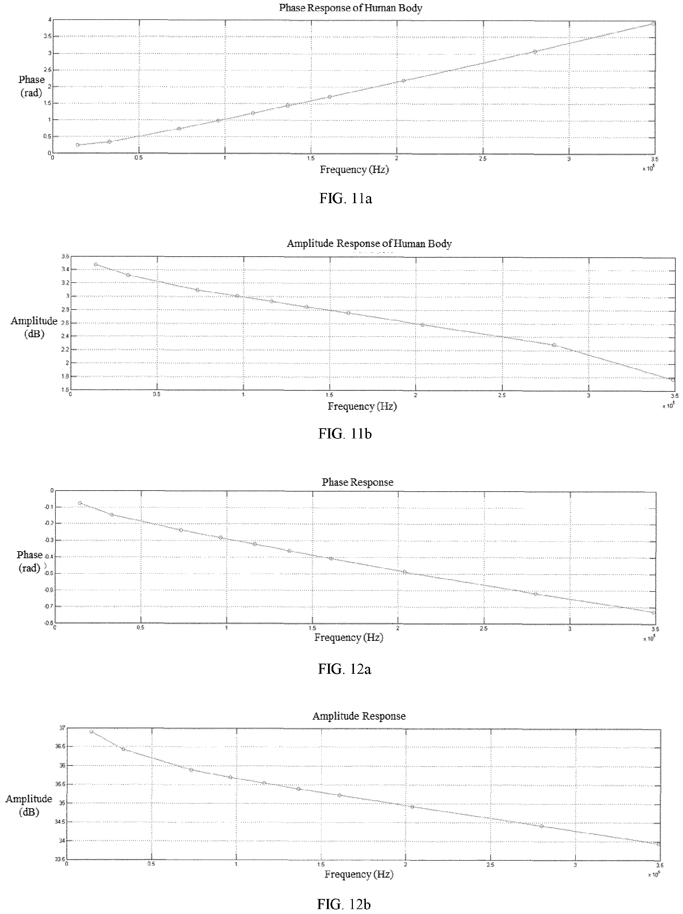

In an alternative embodiment, as shown in FIG. 9, which is an amplitude frequency response diagram. The system receives 10 frequency tones and several interferences, and the interferences have no effect on those 10 frequency tones. The embodiment includes, extracting responses of the 10 frequency tones and obtaining 10 internal frequency tones and 10 external frequency tones in total. The responses of the internal 10 frequency tones are shown in FIG. 10, and the response of the 10 external frequency tones are shown in FIGS. 11a-11b, wherein the external frequency responses include the internal frequency responses.

In an alternative embodiment, schematic diagrams of the external frequency response when removing the internal frequency response are shown in FIGS. 12a-12b. The external resistor network is required in the correction for obtaining the frequency response of the human body.

In an alternative embodiment, the system identification is performed after the frequency responses of the human body system are obtained. However, the system identification is very sensitive to phase noises. Therefore, after obtaining the time sequences of phase and amplitude, the phase noises should be filtered. Human heartbeat is basically 1-2 Hz, so a 10 Hz low-pass filter is appropriate.

5. Separating the frequency domain signals, and filtering the separated frequency domain signals.

Specifically, the system identification is performed after the human multi-chamber model is assumed. If the system is identifiable, the positive coefficients, namely the negative root, of the system transfer function will be obtained. If a positive root or a complex root is received, it means the model is incorrect. The reason is that some tones' interferences are larger, where the interference from each person can occur at a different frequency. Thus the model requires the frequencies of the 10 tunes to be adjustable.

The process of system identification is to solve the minimum error of the measuring point on the frequency response. In an alternative embodiment, measurement on 10 points (frequencies) is employed. If high accuracy is required, frequency points can be added. However, the total energy of the signals is restricted by safety of the human body. Therefore, adding frequency points will reduce the signal-to-noise ratio on each frequency tone.

6. Calculating the resistance and capacitance values for the target tissue and peripheral tissues.

Specifically, the resistances and capacitance values of the target tissue and the peripheral tissues are calculated based on the human body system transfer function. They can be calculated directly from a resistor-capacitor network.

In an alternative embodiment, from the perspective of narrow frequency bands, the tissue's RC value changes little with frequency variation between 10 kHz and 1 MHz. By combining the changes of the two-chamber resistances and capacitances with the electrocardiograph (ECG) timing reference, the cardiovascular state can be estimated.

An alternative embodiment includes, extracting the information from responses of the 10 frequency tones shown in FIG. 9. The information comes from the cardiovascular system and surrounding tissues. A system identification or channel estimation procedure is then executed to calculate the system transfer function. In this example, the system transfer function is:

$$\frac{1.673 * 10^8 S + 4.406 * 10^{13}}{S^2 + 2.939 * 10^6 S + 6.188 * 10^{11}}$$

The two-chamber model calculated from this function is (corrected by 20-Ohm resistance):

60.83 Ohms in parallel with 6.06 nanofarads (nF) and

10.37 Ohms in parallel with 4.23 microfarads ($\mu$F).

The cardiovascular circulatory system and surrounding tissues cannot be distinguished here, and additional information is required to make inferences. In an alternative embodiment, electrocardiograms (ECGs) are used as reference in combination with the location and distribution of the applied electric field. These resistances and capacitances form 4 time sequences. When combining them with electrocardiograms, different information from the cardiovascular circulatory system and surrounding tissues can be separated, and the resistances and capacitances of the cardiovascular system and the surrounding tissues can be obtained.

7. Estimating the state of the target tissue based on the resistance and capacitance values.

Specifically, the resistance and capacitance values are used for multi-chamber modeling, where each chamber consists of a resistor and a capacitor connected in parallel, and multiple chambers are connected in series or in parallel, or in a manner of combination of of serial and parallel connections.

In an alternative embodiment, the two-chamber RC (resistor and capacitor) model is used to simulate the target tissue. A multi-chamber model can be used to simulate the human body. As for thoracic measurement, one chamber represents artery, and/or atria and ventricles, which are main portions of the cardiovascular circulatory system. The other chamber represents the connecting tissue between an electrode and the cardiovascular circulatory system. Each chamber is represented by a parallel RC network composed of integrated resistor and capacitor. The two chambers are connected in series because the heart and the arterial system are not directly connected to the electrodes. The connecting tissues are always between the measuring electrodes and the heart artery. Then, a system identification or channel estimation technology is used to calculate the integrated R (resistance) and C (capacitance) values. The R and C values are used to estimate body fluid, blood flow, and cardiovascular circulatory tissues. The advantage of the two-chamber model is to separate the cardiovascular circulatory system from the surrounding tissues. On the basis of the two-chamber model, there is also a three-chamber model, wherein a parallel RC network is connected in parallel with two parallel RC networks connected in series, as shown in FIG. 4. In an alternative embodiment, the three-chamber model is more close to real human tissues, but the calculation amount is large and the stability is poor.

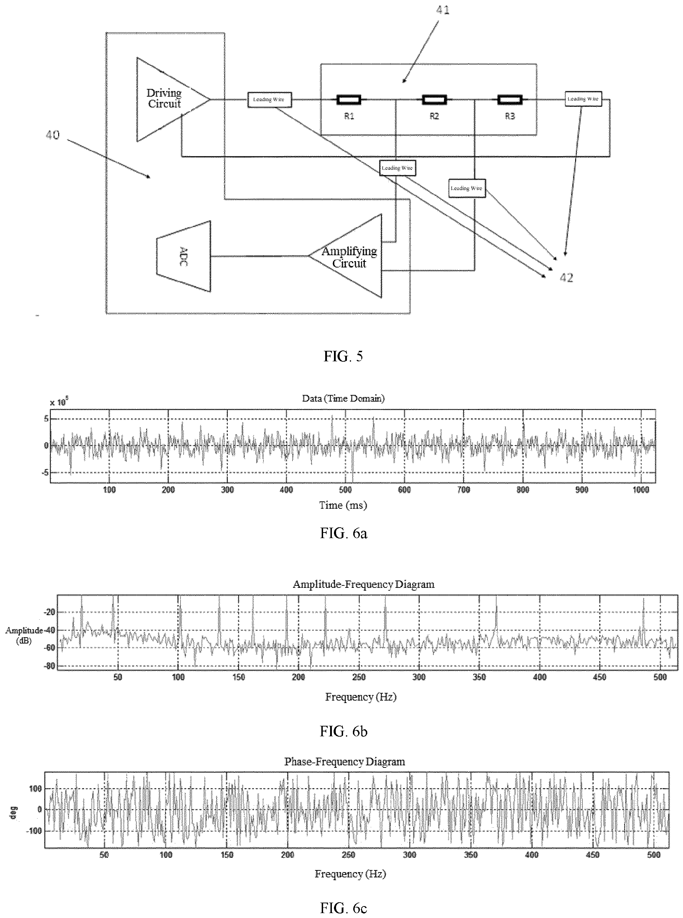

In an alternative embodiment, the phase measurement of the human body system is the most important in the two-chamber or multi-chamber model estimation, which requires hardware to have sufficient bandwidth response and extremely small distortion. Since any system will lead to distortion, the channel estimation is required to detect and counteract these distortions. Distortion will first appear in a driving part, i.e., a circuit for generating the synchronous multi-frequency periodic currents, then in lead wires, finally in a receiving amplifier circuit. In an alternative embodiment, the two-chamber or multi-chamber model further includes a test platform as shown in FIG. 5 to detect distortions. The system firstly measures a resistor network with high accuracy, and then obtains received frequency responses. Since the transmitted current at each frequency is zero-phase, the measured phase response would be the overall distortion of the system. This distortion includes amplitude distortion, which requires correction.

The present invention further provides technologies to measure the R and C values of this two-chamber model. Multiple chamber models can be processed similarly.

In alternative embodiment, 10 frequency responses are provided at a rate of 718 Hz to perform this two-chamber model measurement. These 10 frequency responses are from the demodulation of received signals, and are used to estimate the integrated R and C values. As a result, the two-chamber R and C values are estimated 718 times per second, which is high enough to show the cardiovascular changes. More frequency responses can be used, but more computations would be required.

In an alternative embodiment, in addition to observing, through waveforms, the changes of the resistance and capacitance of the target tissue along with the cardiac cycle so as to prove the correctness of the two-chamber model, some non-invasive experiments can verify some necessary conditions. Table 1 provides four sets of tests to verify the correctness of the two-chamber model. In the first set of the experiments, the measurements were taken under normal conditions; in the second set of experiments, a mixture of salt and water was added under the receiving electrodes, extending 1.5 cm toward the transmitting electrodes; in the third set of experiments, a layer of wet paper towel with Vitamin E, 0.4 mm thick, was added under the transmitting and receiving electrodes; in the fourth set of experiments, the wet paper towel in the third set was replaced by a 4.5 mm thick depilated fresh pig skin. All electrodes are attached to the same marking point. These experiments prove the additivity, that is, when changing the peripheral tissues' resistances and capacitances, the resistances and capacitances of the tissues would not change.

The first normal set of experiments gives reference values. In the second set of experiments, the area of the receiving electrodes expands and approaches the transmitting electrodes, which is equivalent to a larger current in the receiving circuit, therefore the received input voltage becomes larger. As the conductive area of the receiving electrodes increases, the electric field distribution in the peripheral tissues also changes. This would cause changes in the peripheral tissues' resistances and capacitances but little changes in organs. The experimental results proved this inference. In the third set of experiments, it is equivalent to adding a layer of resistors, but the capacitance changes little and both the resistance and the capacitance of the organ should change little. The experimental results well prove this inference.

In the fourth set of experiments, it is equivalent to adding a layer of resistor and capacitor. The results indicate that total resistance increases and the capacitance decreases. Since the contact surface cannot be well matched, the electric field distribution will change, which will greatly affect the measurement results, so the fourth set's results are only instructive. The change ratio of the organ is much smaller than that of the peripheral, even if the absolute value of the organ capacitance is much smaller than that of the peripheral. The three sets of experiments basically proved the additivity of the two-chamber model.

| Testing result / Parameter | First set, normal condition | Second set, 1.5 cm salt water added under the receiving electrodes and extending toward the transmitting electrodes | Third set, (0.4 mm) wet paper towels with Vitamin E added under the transmitting electrodes and receiving electrodes | Fourth set, 4.5 mm depilated fresh pig skin added under the transmitting electrodes and receiving electrodes |
|---|---|---|---|---|
| Peripheral Resistance Rp (Ω) | 5.97±0.018 | 11.54±0.022 | 8.48±0.019 | 18.25±0.028 |
| ΔRp (%) | 0 | 93.30 | 42.04 | 205.70 |
| Organ Resistance Rh (Ω) | 16.7±0.055 | 19.79±0.063 | 17.55±0.052 | 23.97±0.070 |
| ΔRh (%) | 0 | 18.43 | 5.027 | 43.45 |
| Peripheral Capacitance Cp (nF) | 649±3 | 485±2 | 641±3 | 268±0.6 |
| ΔCp (%) | 0 | -25.27 | -1.23 | -58.71 |
| Organ capacitance Ch (nF) | 21.54±0.066 | 19.83±0.060 | 22.92±0.066 | 16.48±0.040 |
| ΔCh (%) | 0 | -7.94 | 6.41 | 23.49 |

Table 1

Another embodiment of the present invention provides a system to implement the above method, wherein the system includes a terminal, an accelerator, and a processor, wherein the terminal includes:

a generator to generate multiple synchronous alternating currents at different frequencies with characteristics of OFDM symbols from frequency domain to time domain, specifically, the generator uses IFFT to generate OFDM symbols and uses the OFDM symbols as a time sequence to generate multiple alternating currents at different frequencies simultaneously;
one or more sensors to transmit multiple generated alternating currents into a human or animal body, and to perceive and receive alternating currents modulated by tissues' changes in the human or animals body;
one or more amplifiers to amplify the modulated alternating currents and digitally convert them into digital signals; and
a pre-processing module to segment the received digital signals into OFDM symbol sequences by using OFDM, to demodulate and separate the digital signals by using FFT, then to filter the separated signals;
the accelerator is configured to calculate FFT or OFDM;
the accelerator is configured to calculate real-time channel estimation and system identification;
the accelerator is configured to calculate related resistance and capacitance values based on the digital signals;
the processor is configured to estimate the states of the tissues by using the resistance and capacitance values;
wherein the processor can be a single computer or multiple computers or a math accelerator array. The terminal also includes processing software and man-machine interfaces that connect human and the system. The computer can be remote, and a person (doctor) can remotely observe the system working in a real-time mode.

Preferably, the present invention provides a system/method to examine the correlation between changes in the body's resistances and capacitances and characteristics of body fluid and cardiovascular circulatory tissues.

The present invention provides a system/method to extract characteristic information from the resistances and the capacitances of the body tissues to represent human hemodynamics and body fluid state, such as the slope values of the resistance and capacitance variation curves, the slope values and time periods of their first derivatives, normalized amplitude change, the integrated shape area and the ratio of different states, but not limited thereto.

The present invention provides a system/method to correlate changes of the target tissue's calculated resistance and capacitance with arterial elasticity.

The present invention provides a system/method to correlate changes of the target tissue's calculated resistance and capacitance with the state of myocardial problems.

The present invention provides a system/method to change the frequency, frequency value, time sequence and intensity of the alternating current.

The present invention provides a system/method to use all the above information to evaluate the health state of cardiovascular circulation, including the state of body fluid.

The foregoing systems and methods can enable accurate detection on a target tissue, with improved detection accuracy.

Embodiments of the present invention now will be described below with reference to the drawings.

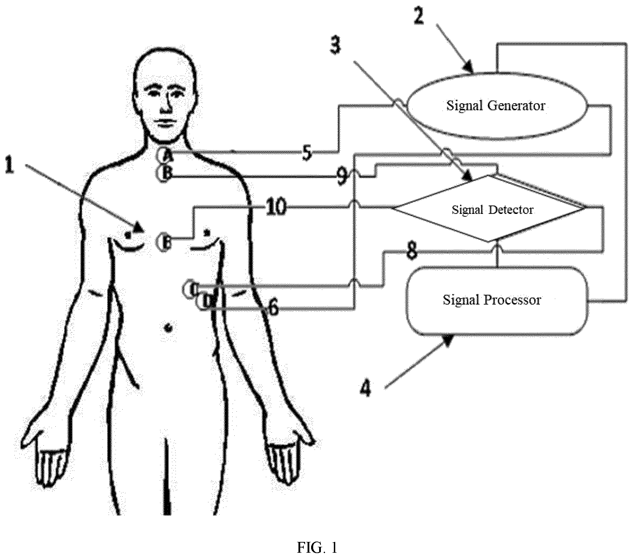

FIG. 1 is a schematic diagram of setting of a terminal system according to an embodiment of the present invention.

Specifically, "1" is a human subject, "A", "B", "C", "D" and "E" are electrodes or contacts connected to a human or animal body (the schematic diagram takes human body as an example); "2" is a signal generator that generates wide band signals composed of multi-frequency components. The generated signals are connected to "A" and "D" by wires or cables "5" and "6". "A" and "D" are selected so that the generated or stimulated signals can pass through related arteries, lungs and the heart, and in this case, pass through the thorax where several major arteries pass through. The signal flow follows the longitudinal direction of the blood flow or the arteries. The generated signals travel inside through the human subject from "A" to "D" or from "D" to "A". "3" is a signal detector, which collects voltage signals from points "B" and "C", "B" and "E", and "E" and "C" through wires or cables "8", "9" and "10". "E" is a special point, which can be composed of a pair of electrodes capable of performing transmission and reception. "4" is a signal processor, which controls and coordinates "2" and "3". "4" also processes signals collected from "B", "C" and "E" and extracts bio-logical information.

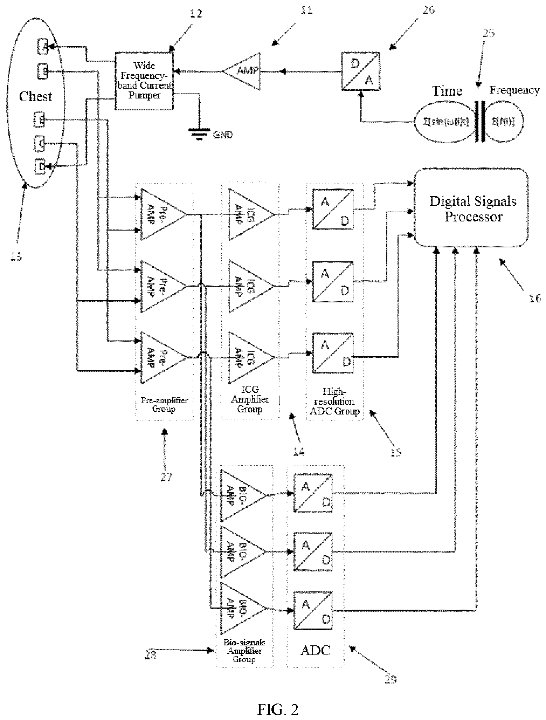

FIG. 2 is a schematic diagram of the function or structure of a terminal system according to another embodiment of the present invention.

Specifically, the terminal can not only acquire the signals, but also send the stimulated currents to human or animal body tissues, wherein "25" is a signal generator that works in both the time domain and the frequency domain. In an alternative embodiment, "25" generates multi-frequency signals. In the time domain, the multi-frequency signals are the summation of multiple sine or cosine waves. In the frequency domain, these signals are the summation of multi-frequency tones. The signal generator "25" transforms the frequency tones into multiple sinusoidal signals in the time domain.

In an alternative embodiment, the generated digital sinusoidal signals pass through a digital-to-analog converter "26", and become analog signals. These analog signals pass through an analog amplifier "11" and get amplified to drive a wide band current pump device "12" and output wide band electrical currents. Starting from the wide band current pump device "12", low-power multiple-frequency sinusoidal waves enter the human body via contacts or electrodes "A" and "D". The human or animal body, which is a complex medium, will modulate the electrical currents. The modulated currents and other bio-electrical signals will be picked up from points "B" and "C", "B" and "E", and "E" and "C".

In an alternative embodiment, since all these signals are weak, they will first be amplified by an analog pre-amplifier group "27". The main function of "27" is to adjust high-impedance input signals, and to convert the high-impedance input signals into low-impedance input signals.

In an alternative embodiment, after "27", the signals enter the impedance cardiogram ICG amplifier through one path, and enter a biological signal amplifier through another path. ICG signals and bio-signals need different gains and filters. Specifically, the ICG signals go into an ICG amplifier group "14". The bio-signals go into a bio-signal amplifier group "28".

In an alternative embodiment, after being amplified, the ICG signals are digitally converted by a high-resolution ADC ("IGC Hi-Res ADC BANK") "15", which is a high-resolution and high-speed analog-to-digital converter bank. The bio-signals are digitized by a low-resolution ADC ("Bio- ADC BANK") "29", which is an analog-to-digital converter bank with low resolution and low sampling rate. The digital signals will be processed by a digital signal processor "16". In an alternative embodiment, the digital signal processor "16" preprocesses the digital signals, such as demodulating, filtering, and extracting different biological signals.

FIG. 3 is a schematic diagram of the structure of a system for performing the method in the present invention according to another embodiment of the present invention.

Specifically, "17" is a path to send the simulated signals. "18" is a path to obtain modulated signals and other biological signals from the human body. In an alternative embodiment, a terminal system "19" performs some pre-processing works, such as demodulation and filtering, but not limited to these tasks. The terminal system "19" can also have its own man-machine interface.

In an alternative embodiment, the terminal system "19" is connected to a math accelerator "21" through "24". The math accelerator "21" is used to perform system identification or channel estimation calculation to obtain RC model values. The intermediate results will be sent to a local computer "20".

In an alternative embodiment, the local computer "20" completes all final processings, such as parameter calculation, feature extraction, and data analysis. In an alternative embodiment, the results and data of the local computer "20"can also be stored in a database server "22", which can be a cloud server. Therefore, the results and data can be retrieved from the local computer "20" and/or the cloud server.

FIG. 4 is a schematic diagram of a measurement circuit of a multi-chamber model according to another embodiment of the present invention.

Specifically, "AC" is a multi-frequency AC current source. L1 and L4 are driving leads that are in contact with the subject. L2 and L3 are receiving leads that are also in contact with the subject. "Zo" is the impedance of a connecting cable. "Cs" is the skin capacitance of the subject. "Rs" is the skin resistance of the subject. "Cp", "Rp", "Cs", "Rs", "Ci" and "Ri" compose a tissue RC model, wherein "Cp" is the capacitance of the peripheral or connecting tissue; "Rp" is the resistance of the peripheral or connecting tissues; "Ci" is the capacitance of the blood circulatory system or interested tissues. "Ri" is the resistance of the blood circulation system or the target tissue; "Cs" is the capacitance of the connecting tissue, which is between the receiving electrode and parallel to the cardiovascular tissue. "Rs" is the resistance of the connecting tissues, which are between the receiving electrodes and parallel to the cardiovascular tissue.

In an alternative embodiment, the multi-chamber model can be simplified into a two-chamber model by removing "Cs" and "Rs", which is formed by two RC chambers connected in series. Using a three-chamber RC model is more close to the real situations, but more calculation amount is required and the stability becomes poor.

FIG. 5 is a schematic diagram of measuring the pure resistance response by using an external resistor network according to another embodiment of the present invention. "40" is a system according to the present invention, "41" is a measured resistor network, and "42" is a lead wire. The system response measured by this platform is the overall response from theoretical transmission to the final actual reception. It includes the system's transmitting and receiving characteristics, as well as the lead wire's characteristics.

FIGS. 6a-6c show the time-domain signals and frequency responses of the system on measuring a resistor network according to another embodiment of the present invention. There are 10 main carriers with different frequencies but the same power, representing 10 frequency tones. They are 14.36KHz, 33.03KHz, 73.24KHz, 96.21KHz, 116.32KHz, 136.42KHz, 160.83KHz, 203.91KHz, 280.02KHz and 348.95KHz respectively. They carry modulation information. FIG. 6a shows a period of a time domain signal received after separation, namely an OFDM symbol. FIG.6b is an amplitude frequency response, which shows some different attenuation caused by the system defects which should be corrected before measuring the human body. FIG. 6c shows the phase frequency response. Since only 10 frequency tones are phase determined, while the others are random, the overall seems to be random.

FIGS. 7a-7b show the waveforms of 10 synchronous frequency tones under theoretical transmission according to another embodiment of the present invention. The phase and amplitude of each frequency tone are controllable and adjustable. FIG. 7a is the result of 1024-point IFFT, which is an OFDM symbol. FIG. 7b is a signal of which the sampling rate is 4 times that of the 1024 points. The signal is repeatedly sent to the digital-to-analog converter (DAC) to generate a periodic analog signal.

FIG. 8 shows the time domain signals actually received by the system according to another embodiment of the present invention. The small amplitude part at the front end is the measured signal of an internal resistor, and the large amplitude sequence behind is the measured signal of the external human body. Each measurement uses the internal frequency response to correct the external frequency response, so as to reduce the random error of the system. Large amplitude pulses in the internal sequence are pseudo ECG data, because the ECG signal is combined into a high-frequency signal.

FIG. 9 shows the amplitude frequency response of a human body test according to another embodiment of the present invention. Its attenuation at the high-frequency end is larger than that of the resistor, indicating that there is a capacitor in the human body.

FIGS. 10a-10b are respectively the amplitude and phase response of the internal resistor under 10 frequency tones according to another embodiment of the present invention. Its phase clearly indicates that the system has phase distortion. This is due to the transmitting circuit and is needed to be corrected. There is a small high-frequency attenuation in the amplitude, which also needs correction.

FIGS. 11a-11b are respectively the amplitude and phase response of the measured human body under 10 frequency tones according to another embodiment of the present invention. The changes are greater than internal changes.

FIGS. 12a-12b are respectively the amplitude and phase response under 10 frequency tones after correction of internal and external resistors according to another embodiment of the present invention. This is the real frequency response of the human body. The amplitude response attenuates more at higher frequencies. The phase response shows more delay at higher frequencies. They indicate that human frequency response is more like an RC system.

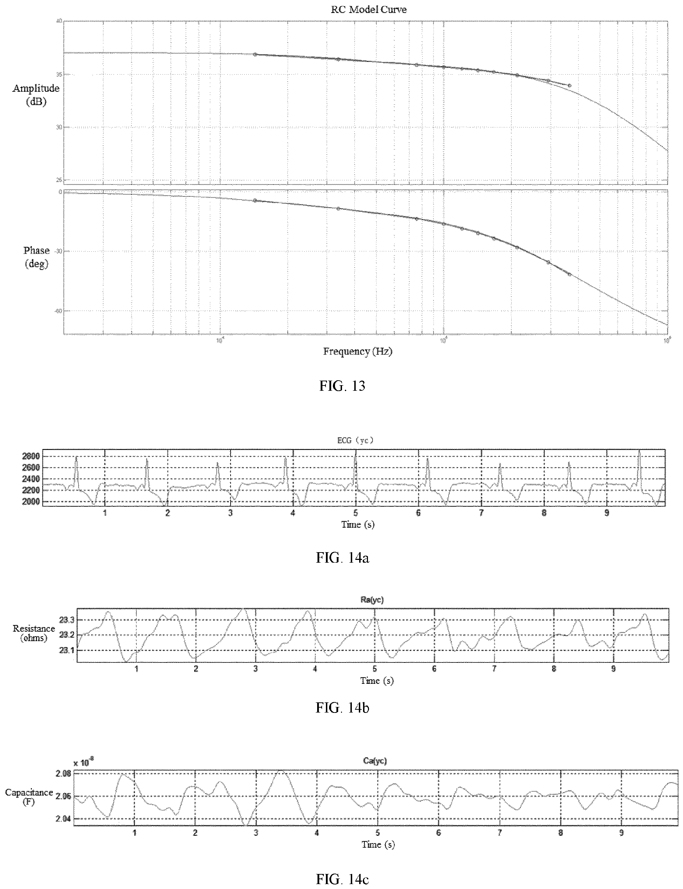

FIG. 13 shows the frequency response (points) of the human body under 10 frequency tones and its system transfer function (line) after system identification according to another embodiment of the present invention. The multi-chamber resistor and capacitor model can be obtained from this system transfer function. It shows the frequency response of the human body with respect to the $2^{nd}$ order RC human body model. The measured frequency response perfectly matches the $2^{nd}$ order RC model. No Cole model behavior is observed here. The reason would be that blood is the main resistor, which may cause the Cole center frequency to be higher. We model the target tissue with a small segmental linear model.

FIGS. 14a-14c are arterial chamber results from a two-chamber model on aorta measurement according to another embodiment of the present invention. Ra is the resistance of the aortic chamber model, and Ca is the capacitance of the aortic chamber model. They follow the heartbeat closely. Before the end diastole, the arteries have the minimal blood reserves, the highest resistance and the lowest capacitance. At the end systole, the arteries have the largest volume, the smallest resistance and the largest capacitance.

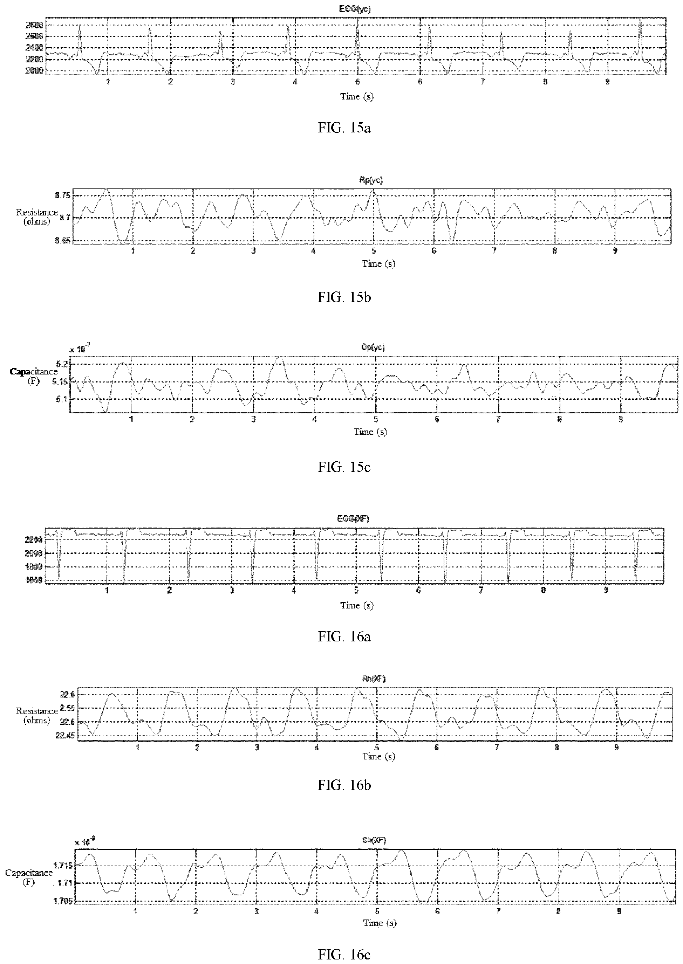

FIGS. 15a-15c are the peripheral chamber results from a two-chamber model on aorta measurement according to another embodiment of the present invention. Rp is the resistance of a peripheral tissue chamber model, and Cp is the capacitance of the peripheral tissue chamber model. They do not show simple rhythmic changes of the heartbeat.

FIGS. 16a-16c are the heart chamber results from a two-chamber model on ventricular measurement according to another embodiment of the present invention. Rh is the resistance of the heart chamber model, and Ch is the capacitance of the heart chamber model. They strongly follow the heartbeat. At the end diastole s, the heart has the most blood, the smallest resistance and the largest capacitance. At the end systole, the heart has the smallest volume, the largest resistance and the smallest capacitance.

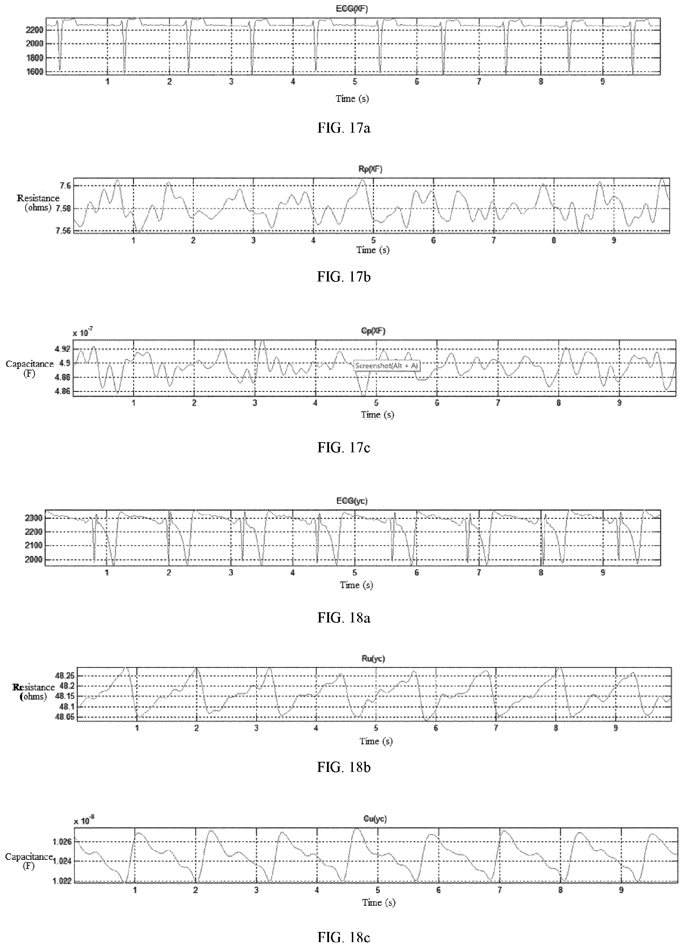

FIGS. 17a-17c are the peripheral chamber results from a two-chamber model on ventricular measurement according to another embodiment of the present invention. Rp is the resistance of the peripheral tissue chamber model, and Cp is the capacitance of the peripheral tissue chamber model. The heartbeat does not change significantly.

FIGS 18a-18c are the arterial chamber results from a two-chamber model on upper chest measurement according to another embodiment of the present invention. Ru is the resistance of the upper chest chamber model, which includes the thoracic artery and heart, and Cu is the capacitance of the upper chest chamber model. They strongly follow the heartbeat. The arteries have the minimal blood reserves, the highest resistance and the lowest capacitance before ventricular compression. At the end systoles, the arteries have the largest volume, the smallest resistance and the largest capacitance.

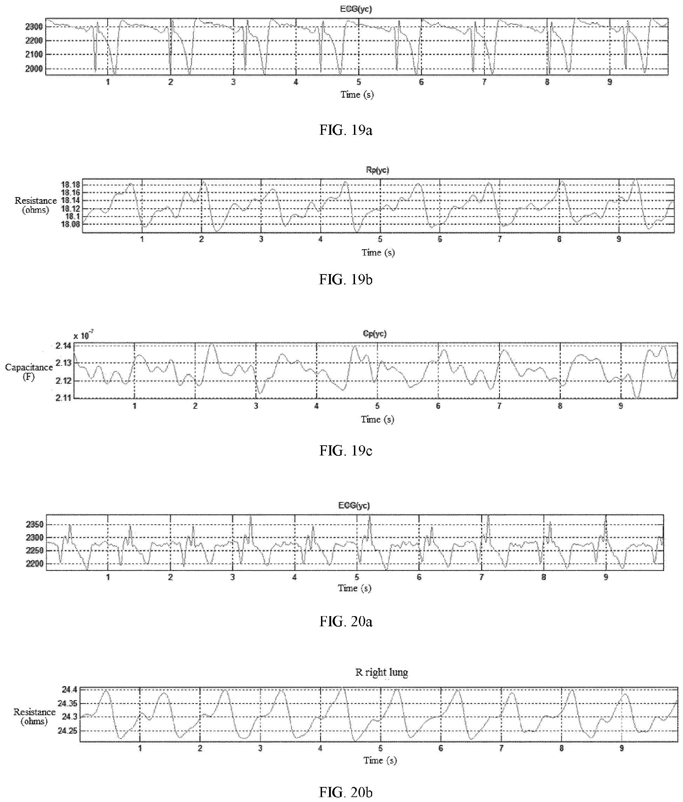

FIGS. 19a-19c are the peripheral chamber results from a two-chamber model on upper chest measurement according to another embodiment of the present invention. Rp is the resistance of the peripheral tissue chamber model, and Cp is the capacitance of the peripheral tissue chamber model. They don't change as clearly as in the upper chest model.

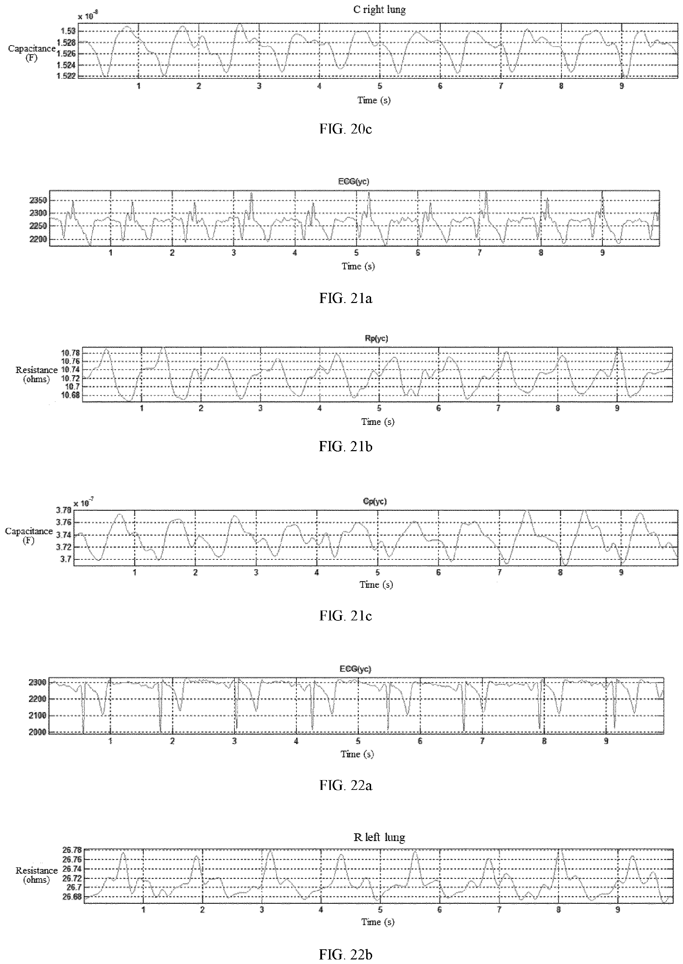

FIGS. 20a-20c are the artery/vein chamber results from a two-chamber model on right lung measurement according to another embodiment of the present invention. "R right lung" is the resistance of the right lung's artery/vein chamber model, and "C right lung" is the capacitance of the right lung's artery/vein chamber model. They vary along with the heartbeat.

FIGS. 21a-21c are the peripheral chamber results from a two-chamber model on right lung measurement according to another embodiment of the present invention. Rp is the resistance of the right lung's peripheral tissue chamber model, and Cp is the capacitance of the right lung's peripheral tissue chamber model. They change along with the heartbeat.

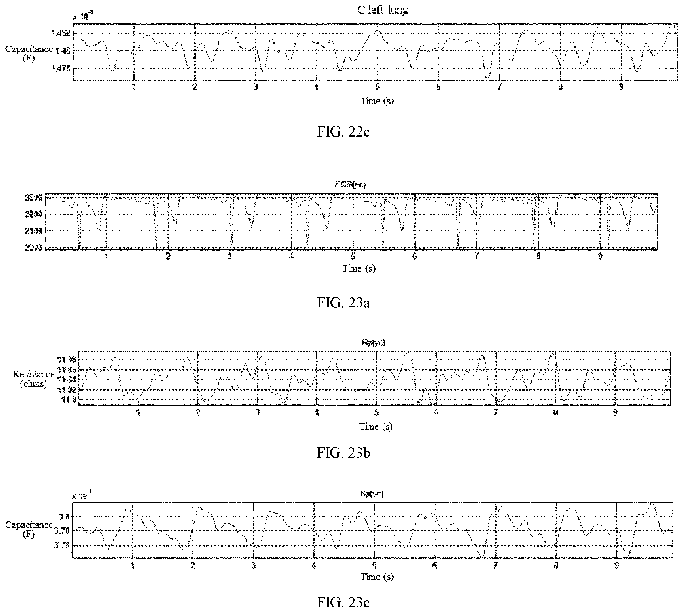

FIGS. 22a-22c are the artery/vein chamber results from a two-chamber model on left lung measurement according to another embodiment of the present invention. "R left lung" is the resistance of the left lung's artery/vein chamber model, and "C left lung" is the capacitance of the left lung's artery/vein chamber model. They vary along with the heartbeat.

FIG. 23a-23c are the of the peripheral chamber results from the two-chamber model on left lung measurement according to another embodiment of the present invention. Rp is the resistance of the left lung's peripheral tissue chamber model, and Cp is the capacitance of the left lung's peripheral tissue chamber model. They change along with the heartbeat. This application provides a method and system to detect characteristic information of body tissues, which applies multiple alternating currents at different frequencies to the human body simultaneously. After receiving the modulated voltage signals, the system demodulates the received signals and extracts information of the cardiovascular system and surrounding tissues from carriers at specified frequencies. By performing system identification or channel estimation procedures, the information is separated from the cardiovascular circulatory system and surrounding tissues. The resistances and capacitances of the cardiovascular system and surrounding tissues are calculated respectively, and the states of body fluid and cardiovascular circulation are represented by the calculated resistances and capacitances. As a result, the method and system can accurately and reliably obtain the corresponding state information, and accurately measure the target tissue to obtain its health state.

The technical scope of the present invention should be determined according to the scope of the claims.

**Claims**

1. A non-invasive method to detect characteristic information of body tissues, wherein is the method is used to capture changes in body fluid, blood flow, and/or cardiovascular circulatory tissues, the method comprising:

   generating multiple synchronous alternating currents at different frequencies simultaneously with characteristics of Orthogonal Frequency Division Multiplexing symobols, OFDM symbols, by using Inverse Fast Fourier transform, IFFT, and transmitting them to a human or animal body;
   receiving the alternating currents modulated by tissues and their changes in the human or animal body;
   amplifying the modulated alternating currents and digitally converting them into digital signals;
   pre-processing the digital signals, the pre-processing further comprising: segmenting the digital signals into OFDM symbol sequences, demodulating the OFDM symbol sequences by using FFT to obtain frequency domain signals; and
   estimating a state of a target tissue based on frequency domain signals,
   **characterised in that** estimating the state of a target tissue based on the frequency domain signals comprises:

   separating the frequency domain signals and filtering the separated frequency domain signals;
   calculating the resistance and capacitance values of the target tissue and peripheral tissues; and
   estimating the state of the target tissue based on the resistance and capacitance values, wherein the estimating the state of the target tissue based on the resistance and capacitance values comprises: performing multi-chamber modeling based on the resistance and capacitance values, where each chamber consists of a resistor and a capacitor connected in parallel, and the multiple chambers are connected in series, or in parallel, or in a manner of combination of serial and parallel connections.

2. The non-invasive method to detect characteristic information of body tissues according to claim 1, wherein the generating multiple synchronous alternating currents at different frequencies simultaneously by using IFFT, which converts signals from the frequency domain to the time domain, wherein the alternating currents at different frequencies are periodic, and the intensity, phase, and/or frequency of the alternating currents are adjustable.

3. The non-invasive method to detect characteristic information of body tissues according to claim 1, wherein the receiving the alternating currents modulated by tissues' changes in the human or animal body comprises: determining a period of the alternating currents, wherein the period is an OFDM symbol length, and synchronously receiving the modulated alternating signals in each period.

4. The non-invasive method to detect characteristic information of body tissues according to claim 1, wherein the separating the frequency domain signals comprising: based on the amplitudes and phases of the frequency domain signals, calculating a human body system transfer function through system identification and channel estimation methods.

5. The non-invasive method to detect characteristic information of body tissues according to claim 4, wherein the calculating the resistance and capacitance values of the target tissue and peripheral tissues comprises: calculating the resistance and capacitance values of the target tissue and peripheral tissues based on the human body system transfer function.

6. The non-invasive method to detect characteristic information of body tissues according to claim 1, wherein the frequency range is from 10KHz to 1MHz.

7. The non-invasive method to detect characteristic information of body tissues according to claim 1, wherein the multi-chamber modeling is two-chamber modeling, wherein a connecting tissue is between an electrode and the target tissue.

8. A system to implement any of the methods above, wherein the system comprises a terminal and a processor, wherein the terminal comprises:

a generator, configured to generate multiple synchronous alternating currents at different frequencies with OFDM symbols characteristics by using IFFT;

one or more sensors, configured to transmit the multiple alternating currents into a human or animal body and to receive the alternating currents modulated by tissues' changes in the human or animal body;

one or more amplifiers, configured to amplify the modulated alternating currents and digitally convert them into digital signals;

a pre-processing module, configured to segment the digital signals into OFDM symbol sequences, to demodulate OFDM symbols sequences by using FFT and to obtain the frequency domain signals, **characterised in that** the pre-processing module is further configured to separate the frequency domain signals and filter the separated frequency domain signals;

wherein the processor is configured to calculate resistance and capacitance values and estimate the state of a target tissue based on the resistance and capacitance values,

wherein the processor is configured to establish an equivalent circuit of multiple chambers through the resistance and capacitance values, each chamber consists of a resistor and a capacitor connected in parallel, and the multiple chambers are connected in series, or in parallel, or in a manner of combination of serial and parallel connections.

9. The system according to claim 8, wherein the generator is configured to generate the multiple alternating currents at different frequencies, wherein the alternating currents at different frequencies are periodic, and the intensity, phase and/or frequency of the alternating currents are adjustable.

10. The system according to claim 8, wherein the processor is configured to determine a period of the alternating currents, wherein the period is an OFDM symbol length, and to receive the modulated alternating signals synchronously at each of the period.

11. The system according to claim 10, wherein the separation comprises: based on the amplitudes and phases of the frequency domain signals, calculating a human body system transfer function through system identification and channel estimation methods .

12. The system according to claim 11, wherein the system further comprising an accelerator, the accelerator is configured to calculate the resistance and capacitance values of the target tissue and peripheral tissues, and the calculation comprises calculations based on the human body system transfer function.

13. The system according to any of claims 8 to 12, wherein the terminal further comprises a human-machine interface configured to operate the system and/or display results.

14. The system according to any one of claims 8 to 13, wherein the system comprises a database for storing processed results and data of the processor and the processor is configured to retrieve the database.

15. The system of claim 14, wherein the processor is remote and configured to remotely observe the system working in a real-time mode.

**Patentansprüche**

1. Nicht-invasives Verfahren zum Erfassen charakteristischer Informationen von Körpergeweben, wobei das Verfahren verwendet wird, um Veränderungen in Körperflüssigkeit, Blutfluss und/oder kardiovaskulärem Kreislaufgewebe zu erfassen, wobei das Verfahren Folgendes umfasst:

Erzeugung mehrerer synchroner Wechselströme bei unterschiedlichen Frequenzen gleichzeitig mit den Eigenschaften der orthogonalen Frequenzteilung Multiplexing-Symbole, OFDM-Symbole, unter Verwendung der Inverse Fast Fourier Transform, IFFT, und deren Übertragung auf einen menschlichen oder tierischen Körper;

Empfangen von Wechselströmen, die durch Gewebe und deren Veränderungen im menschlichen oder tierischen Körper moduliert werden;

Verstärkung der modulierten Wechselströme und digitale Umwandlung in digitale Signale;

Vorverarbeitung der digitalen Signale, wobei die Vorverarbeitung ferner umfasst:

Segmentierung der digitalen Signale in OFDM-Symbolsequenzen, Demodulation der OFDM-Symbolsequenzen durch Verwendung von FFT zur Gewinnung von Frequenzbereichssignalen; und

Schätzung eines Zustands eines Zielgewebes auf der Grundlage von Signalen im Frequenzbereich, **dadurch gekennzeichnet, dass**

die Schätzung des Zustands eines Zielgewebes auf der Grundlage der Signale im Frequenzbereich Folgendes umfasst:

Trennung der Frequenzbereichssignale und Filterung der getrennten Frequenzbereichssignale;
Berechnung der Widerstands- und Kapazitätswerte des Zielgewebes und des peripheren Gewebes; und

Schätzung des Zustands des Zielgewebes auf der Grundlage der Widerstands- und Kapazitätswerte, wobei die Schätzung des Zustands des Zielgewebes auf der Grundlage der Widerstands- und Kapazitätswerte umfasst: Durchführung einer Mehrkammermodellierung auf der Grundlage der Widerstands- und Kapazitätswerte, wobei jede Kammer aus einem Widerstand und einem Kondensator besteht, die parallel geschaltet sind, und die mehreren Kammern in Reihe oder parallel oder in einer Kombination aus seriellen und parallelen Verbindungen geschaltet sind.

2. Nicht-invasives Verfahren zum Erfassen charakteristischer Informationen von Körpergeweben nach Anspruch 1, wobei das Erzeugen mehrerer synchroner Wechselströme bei unterschiedlichen Frequenzen gleichzeitig unter Verwendung von IFFT, das Signale aus dem Frequenzbereich in den Zeitbereich umwandelt, wobei die Wechselströme bei unterschiedlichen Frequenzen periodisch sind und die Intensität, Phase und/oder Frequenz der Wechselströme einstellbar sind.

3. Nicht-invasives Verfahren zum Erfassen charakteristischer Informationen von Körpergeweben nach Anspruch 1, wobei das Empfangen der Wechselströme, die durch Gewebeveränderungen im menschlichen oder tierischen Körper moduliert, Folgendes umfasst: Bestimmen einer Periode der Wechselströme, wobei die Periode eine OFDM-Symbollänge ist, und synchrones Empfangen der modulierten Wechselsignale in jeder Periode.

4. Nicht-invasives Verfahren zum Erfassen charakteristischer Informationen von Körpergeweben nach Anspruch 1, wobei das Trennen der Frequenzbereichssignale Folgendes umfasst: Berechnung einer Systemübertragungsfunktion des menschlichen Körpers durch Systemidentifikations- und Kanalschätzungsmethoden basierend auf den Amplituden und Phasen der Signale im Frequenzbereich.

5. Nicht-invasives Verfahren zum Erfassen charakteristischer Informationen von Körpergeweben nach Anspruch 4, wobei die Berechnung der Widerstands- und Kapazitätswerte des Zielgewebes und peripherer Gewebe Folgendes umfasst: Berechnung der Widerstands- und Kapazitätswerte des Zielgewebes und der peripheren Gewebe auf der Grundlage der Transferfunktion des menschlichen Körpersystems.

6. Nicht-invasives Verfahren zum Erfassen charakteristischer Informationen von Körpergeweben nach Anspruch 1, wobei der Frequenzbereich von 10KHz bis 1MHz liegt.

7. Nicht-invasives Verfahren zum Erfassen charakteristischer Informationen von Körpergeweben nach Anspruch 1, wobei die Mehrkammermodellierung eine Zweikammermodellierung ist, wobei sich ein Verbindungsgewebe zwischen einer Elektrode und dem Zielgewebe befindet.

8. System zum Implementieren eines der oben genannten Verfahren, wobei das System ein Terminal und einen Prozessor umfasst, wobei das Terminal Folgendes umfasst:

einen Generator, der so konfiguriert ist, dass er mehrere synchrone Wechselströme mit unterschiedlichen Frequenzen mit OFDM-Symboleigenschaften unter Verwendung von IFFT erzeugt;
einen oder mehrere Sensoren, die so konfiguriert sind, dass sie die mehreren Wechselströme in einen menschlichen oder tierischen Körper übertragen und die Wechselströme empfangen, die durch Veränderungen des Gewebes im menschlichen oder tierischen Körper moduliert werden;
einen oder mehrere Verstärker, die so konfiguriert sind, dass sie die modulierten Wechselströme verstärken und digital in digitale Signale umwandeln;
ein Vorverarbeitungsmodul, das für die Segmentierung der digitalen Signale in OFDM-Symbolsequenzen, für die Demodulation von OFDM-Symbolsequenzen unter Verwendung von FFT und für die Gewinnung von Frequenzbereichssignalen konfiguriert ist, **dadurch gekennzeichnet, dass**

das Vorverarbeitungsmodul ferner so konfiguriert ist, dass es die Signale im Frequenzbereich trennt und die Signale im getrennten Frequenzbereich filtert,

wobei der Prozessor so konfiguriert ist, dass er Widerstands- und Kapazitätswerte berechnet und den Zustand eines Zielgewebes auf der Grundlage der Widerstands- und Kapazitätswerte schätzt,

wobei der Prozessor so konfiguriert ist, dass er eine Ersatzschaltung aus mehreren Kammern durch die Widerstands- und Kapazitätswerte herstellt, jede Kammer aus einem Widerstand und einem Kondensator besteht, die parallel geschaltet sind, und die mehreren Kammern in Reihe oder parallel oder in einer Kombination von seriellen und parallelen Verbindungen geschaltet sind.

9. System nach Anspruch 8, wobei der Generator so konfiguriert ist, dass er die mehreren Wechselströme bei unterschiedlichen Frequenzen erzeugt, wobei die Wechselströme bei unterschiedlichen Frequenzen periodisch sind und die Intensität, Phase und/oder Frequenz der Wechselströme einstellbar sind.

10. System nach Anspruch 8, wobei der Prozessor so konfiguriert ist, dass er eine Periode der Wechselströme bestimmt, wobei die Periode eine OFDM-Symbollänge ist, und die modulierten Wechselsignale synchron zu jeder der Perioden empfängt.

11. System nach Anspruch 10, wobei die Trennung umfasst: Basierend auf den Amplituden und Phasen der Signale im Frequenzbereich, Berechnung einer Systemübertragungsfunktion des menschlichen Körpers durch Systemidentifikations- und Kanalschätzungsmethoden.

12. System nach Anspruch 11, wobei das System weiterhin einen Beschleuniger umfasst, wobei der Beschleuniger so konfiguriert ist, dass er die Widerstands- und Kapazitätswerte des Zielgewebes und der peripheren Gewebe berechnet, und die Berechnung Berechnungen umfasst, die auf der Übertragungsfunktion des Systems des menschlichen Körpers basieren.

13. System nach einem der Ansprüche 8 bis 12, wobei das Terminal weiterhin eine Mensch-Maschine-Schnittstelle umfasst, die konfiguriert ist, um das System zu bedienen und/oder Ergebnisse anzuzeigen.

14. System nach einem der Ansprüche 8 bis 13, wobei das System eine Datenbank zum Speichern verarbeiteter Ergebnisse und Daten des Prozessors umfasst und der Prozessor so konfiguriert ist, dass er die Datenbank abruft.

15. System nach Anspruch 14, wobei der Prozessor entfernt und so konfiguriert ist, dass er das System in einem Echtzeitmodus fernbeobachtet.

**Revendications**

1. Procédé non invasif pour détecter des informations caractéristiques de tissus corporels, dans lequel le procédé est utilisé pour capturer des modifications dans les fluides du corps, le flux sanguin et/ou les tissus circulatoires cardiovasculaires, le procédé comprenant :

générer simultanément plusieurs courants alternatifs synchrones à différentes fréquences ayant des caractéristiques de symboles de multiplexage à division orthogonale de fréquence, soit symboles OFDM, en utilisant la transformation de Fourier rapide inverse, soit IFFT, et en les transmettre vers un corps humain ou animal ;
recevoir les courants alternatifs modulés par les tissus et leurs variations dans le corps humain ou animal ;
amplifier les courants alternatifs modulés et les convertir numériquement en signaux numériques ;
pré-traiter les signaux numériques, le pré-traitement comprenant en outre : segmenter les signaux numériques en séquences de symboles OFDM, démoduler les séquences de symboles OFDM en utilisant la FFT pour obtenir des signaux de domaine fréquentiel ; et
estimer un état d'un tissu cible en fonction des signaux de domaine fréquentiel, **caractérisée en ce que**, l'étape d'estimer l'état d'un tissu cible en fonction des signaux de domaine fréquentiel comprend :

séparer les signaux de domaine fréquentiel et filtrer les signaux de domaine fréquentiel séparés ;
calculer les valeurs de résistance et de capacité du tissu cible et des tissus périphériques ; et
estimer l'état du tissu cible en fonction des valeurs de résistance et de capacité, l'étape d'estimer l'état du tissu cible en fonction des valeurs de résistance et de capacité comprenant : effectuer une modélisation multichambre en fonction des valeurs de résistance et de capacité, dans laquelle chaque chambre comprend une résistance et un condensateur connectés en parallèle, et les multiples chambres sont connectées en série ou en parallèle, ou d'une manière de combinaison de connexions en série et en parallèle.

2.  Procédé non invasif pour détecter des informations caractéristiques de tissus corporels selon la revendication 1, dans lequel l'étape de générer simultanément plusieurs courants alternatifs synchrones à différentes fréquences en utilisant l'IFFT consiste à convertir les signaux de domaine fréquentiel en signaux de domaine temporel, les courants alternatifs à différentes fréquences étant périodiques, et l'intensité, la phase et/ou la fréquence des courants alternatifs étant réglables.

3.  Procédé non invasif pour détecter des informations caractéristiques de tissus corporels selon la revendication 1, dans lequel la réception des courants alternatifs modulés par les variations du tissu dans le corps humain ou animal comprend : déterminer une période des courants alternatifs, la période étant une longueur de symbole OFDM, et recevoir synchroniquement les signaux alternatifs modulés pendant chaque période.

4.  Procédé non invasif pour détecter des informations caractéristiques de tissus corporels selon la revendication 1, dans lequel la séparation des signaux de domaine fréquentiel comprend : calculer, en fonction des amplitudes et des phases des signaux de domaine fréquentiel, une fonction de transfert de système du corps humain par des méthodes d'identification de système et d'estimation de canal.

5.  Procédé non invasif pour détecter des informations caractéristiques de tissus corporels selon la revendication 4, dans lequel l'étape de calculer les valeurs de résistance et de capacité du tissu cible et des tissus périphériques comprend : calculer les valeurs de résistance et de capacité du tissu cible et des tissus périphériques en fonction de la fonction de transfert de système du corps humain.

6.  Procédé non invasif pour détecter des informations caractéristiques de tissus corporels selon la revendication 1, dans lequel la plage de fréquence est de 10KHz à 1MHz.

7.  Procédé non invasif pour détecter des informations caractéristiques de tissus corporels selon la revendication 1, dans lequel la modélisation multichambre est une modélisation à deux chambres, un tissu de connexion se trouvant entre une électrode et le tissu cible.

8.  Système de mise en oeuvre du procédé ci-dessus, dans lequel le système comprend un terminal et un processeur, dans lequel le terminal comprend :

    un générateur, configuré pour générer plusieurs courants alternatifs synchrones à différentes fréquences ayant des caractéristiques de symboles OFDM en utilisant l'IFFT ;
    un ou plusieurs capteurs, configurés pour transmettre les plusieurs courants alternatifs vers un corps humain ou animal et recevoir les courants alternatifs modulés par les variations du tissu dans le corps humain ou animal ;
    un ou plusieurs amplificateurs, configurés pour amplifier les courants alternatifs modulés et les convertir numériquement en signaux numériques ;
    un module de pré-traitement, configuré pour segmenter les signaux numériques en séquences de symboles OFDM, démoduler les séquences de symboles OFDM en utilisant la FFT et obtenir des signaux de domaine fréquentiel ; **caractérisé en ce que**,
    le module de pré-traitement est configuré en outre pour séparer les signaux de domaine fréquentiel et filtrer les signaux de domaine fréquentiel séparés ;
    dans lequel le processeur est configuré pour calculer des valeurs de résistance et de capacité et estimer l'état du tissu cible en fonction des valeurs de résistance et de capacité,
    dans lequel le processeur est configuré pour établir un circuit équivalent de plusieurs chambres sur la base des valeurs de résistance et de capacité, chaque chambre comprenant une résistance et un condensateur connectés en parallèle, et les multiples chambres étant connectées en série ou en parallèle, ou d'une manière de combinaison de connexions en série et en parallèle.

9.  Système selon la revendication 8, dans lequel le générateur est configuré pour générer les plusieurs courants alternatifs à différentes fréquences, les courants alternatifs à différentes fréquences étant périodiques, et l'intensité, la phase et/ou la fréquence des courants alternatifs étant réglables.

10. Système selon la revendication 8, dans lequel le processeur est configuré pour déterminer une période des courants alternatifs, la période étant une longueur de symbole OFDM, et recevoir synchroniquement les signaux alternatifs modulés pendant chaque période.

11. Système selon la revendication 10, dans lequel la séparation comprend : calculer, en fonction des amplitudes et des phases des signaux de domaine fréquentiel, une fonction de transfert de système du corps humain par des méthodes d'identification de système et d'estimation de canal.

12. Système selon la revendication 11, dans lequel le système comprend en outre un accélérateur, l'accélérateur étant configuré pour calculer les valeurs de résistance et de capacité du tissu cible et des tissus périphériques, et le calcul consistant à calculer en fonction de la fonction de transfert de système du corps humain.

13. Système selon l'une quelconque des revendications 8 à 12, dans lequel le terminal comprend en outre une interface homme-machine pour commander le système et/ou afficher les résultats.

14. Système selon l'une quelconque des revendications 8 à 13, dans lequel le système comprend une base de données pour stocker des résultats de traitement et des données du processeur, et le processeur est configuré pour récupérer la base de données.

15. Système selon la revendication 14, dans lequel le processeur est commandé à distance et est configuré pour observer à distance le fonctionnement du système en mode temps réel.

FIG. 1

FIG. 2

FIG. 3

FIG. 4

FIG. 5

FIG. 6a

FIG. 6b

FIG. 6c

FIG. 7a

FIG. 7b

FIG. 8

23

FIG. 9

FIG. 10a

FIG. 10b

Phase Response of Human Body

FIG. 11a

Amplitude Response of Human Body

FIG. 11b

Phase Response

FIG. 12a

Amplitude Response

FIG. 12b

FIG. 13

FIG. 14a

FIG. 14b

FIG. 14c

FIG. 15a

FIG. 15b

FIG. 15c

FIG. 16a

FIG. 16b

FIG. 16c

27

ECG(XF)

FIG. 17a

Rp(XF)

FIG. 17b

Cp(XF)

FIG. 17c

ECG(yc)

FIG. 18a

Ru(yc)

FIG. 18b

Cu(yc)

FIG. 18c

ECG(yc)

FIG. 19a

Rp(yc)

FIG. 19b

Cp(yc)

FIG. 19c

ECG(yc)

FIG. 20a

R right lung

FIG. 20b

FIG. 20c

FIG. 21a

FIG. 21b

FIG. 21c

FIG. 22a

FIG. 22b

FIG. 22c

FIG. 23a

FIG. 23b

FIG. 23c

**EP 3 957 240 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011158166 A1
- US 20180206759 A1
- US 20180067063 A1
- EP 1138259 A2
- US 6339722 B1